# EUROPEAN PATENT APPLICATION

(11) **EP 1 630 556 A1**
(43) Date of publication of application: **01.03.2006**
(21) Application number: 04736124.1
(22) Date of filing: 04.06.2004
(51) Int. Cl.: G01N 33/53, G01N 37/00

(54) **BIOCHIP SUBSTRATE**

(30) Priority: 05.06.2003 JP 2003160886
(71) Applicant: ASAHI GLASS COMPANY LTD., Tokyo 100-8405 (JP)
(72) Inventor: NOMOTO, Hideo, c/o Asahi Glass Company, Limited, Yokohama-shi, Kanagawa 221-8755 (JP); TOHDA, Hideki, c/o Asahi Glass Company, Limited, Yokohama-shi, Kanagawa 221-8755 (JP)
(74) Representative: Müller-Boré & Partner Patentanwälte
(86) International application number: PCT/JP2004/008156
(87) International publication number: WO 2004/109281

(57) **Abstract**

The object is to provide a biochip substrate which is suitable for high density and high precision immobilization of DNA probes by stamping and for fabrication of a biochip which secures a high S/N ratio and high spot homogeneity at each spot without blurring or other problems when used. A biochip substrate having a porous layer which contains inorganic particles such as silica particles or alumina particles and preferably has an average pore radius of from 1 to 100 nm and a pore volume of 0.1 to 5 cm³/g, on a base.

## Description

### TECHNICAL FIELD

The present invention relates to a substrate suitable to make a biochip by arranging and immobilizing trace amounts of biological high-molecular-weight oligomers such as DNA, RNA, sugar chains and proteins corresponding to hundreds to tens thousands of genes.

### BACKGROUND ART

Among biochips, typical are DNA chips having from hundreds to thousands of microspots of numerous DNA fragments (hereinafter referred to as DNA probes) immobilized on the substrate. DNA chips are reacted (hybridized) with DNA from human or animals (hereinafter referred to as DNA analytes) to be examined for numerous DNA sequences to analyze sequence variation among individuals, gene expression in cells in different states and the like. The subsequent description will deal with DNA as a representative, though it also applies to RNA, proteins and sugar chains.

Biochip fabrication is roughly classified on the basis of the method of immobilization of DNA probes on the substrate, as the photolithographic synthesis on a solid phase and as the array stamping of numerous kinds of pre-synthesized DNA probes on the substrate. Photolithographic synthesis on a solid phase is unavailable for molecules having higher-order structures like proteins and unsuitable for cost reduction, though this technique allows synthesis and arrangement of various biological high-molecular-weight oligomers at high density (JP-A-2002-131327).

On the other hand, the stamping technique comprises depositing solutions containing DNA probes to predetermined positions on a substrate surface somehow and is considered to be suited to reduce costs without many restrictions on DNA probes. As concrete stamping means, pin transfer (JP-A-2000-157272) and ink jetting (JP-A-2001-324505) have been proposed.

Conventional biochip substrates include glass plates such as glass slides having porous polymers such as nitrocellulose thereon (US Patent 6,319,674), glass substrate having a coating of polylysine containing amino and carboxyl groups on the surface (Science, America, 1995, vol.270, p.467-470) and glass substrates having an aminosilane coating (Nucleic Acids Research, UK, 1994, vol.22, p.5456-5465). It is also known to immobilize synthetic DNA fragments on a substrate via covalent bonds with functional groups such as amino groups previously introduced at the ends of the DNA fragments (JP-A-2003-121437). However, in these cases, there are problems of inhomogeneity or blurring, and no biochip substrates suitable to make a biochip with a high S/N ratio at each spot by stamping have been proposed.

### DISCLOSURE OF THE INVENTION

It is an object of the present invention to provide a biochip substrate which is suitable for high density and high precision immobilization of DNA probes by stamping and for fabrication of a biochip which secures a high S/N ratio and high spot homogeneity at each spot without blurring or other problems when used.

The present invention provides the following.
(1) A biochip substrate comprising a base and a porous layer containing inorganic particles provided on the base.
(2) The biochip substrate according to (1), wherein the inorganic particles are alumina particles and/or silica particles.
(3) The biochip substrate according to (2), wherein the inorganic particles are boehmite particles.
(4) The biochip substrate according to (1), (2) or (3), wherein the porous layer has an average pore radius of from 1 to 100 nm.
(5) The biochip substrate according to (1), (2), (3) or (4), wherein the porous layer has a pore volume of 0.1 to 5 cm³/g.
(6) The biochip according to (1), (2), (3), (4) or (5), which has a layer containing a DNA fragment on the surface of the porous layer.

### BRIEF DESCRIPTION OF THE DRAWINGS

Fig. 1(a) is a (two-dimensional) illustration of the fluorescence from a spot on a boehmite substrate, and Fig. 1(b) is a (two-dimensional) illustration of the fluorescence from a spot on a comparative substrate.
Fig. 2 is a (three-dimensional) illustration of the fluorescence from a spot on a boehmite substrate.
Fig. 3 is a (three-dimensional) illustration of the fluorescence from a spot on a comparative substrate.

### BEST MODE FOR CARRYING OUT THE INVENTION

The biochip substrate of the present invention (hereinafter referred to as the present substrate) is characterized by having a porous layer containing inorganic particles on a base. In the present invention, the base may be a glass, synthetic quartz glass, ceramic, metal or plastic plate, film or the like, though it is not particularly restricted. DNA probes are usually labeled with a fluorophor. The less the fluorescence from outside of the fluorophor (hereinafter referred to as the background fluorescence) is, the higher the detection sensitivity is. In this sense, glass, synthetic quartz glass, ceramics and metals are preferred.

The inorganic particles constituting the porous layer may be oxide particles such as alumina particles, silica particles, zirconia particles and mullite particles, non-oxide particles such as silicon carbide particles and silicon nitride particles, or metal particles such as silicon particles.

The inorganic particles in the present substrate are preferably oxide particles, particularly preferably alumina particles and/or silica particles, to immobilize DNA probes firmly. The reason, though not known in detail, seems to be that when fluorescently labeled DNA probes carry charges, alumina particles and silica particles having charged surfaces can hold them firmly through electrostatic bonding. Because DNA probes are usually negatively charged, it is particularly preferred that the inorganic particles are alumina particles.

The alumina particles are preferably boehmite (alumina hydrate) particles to firmly immobilize DNA probes. Herein, boehmite means alumina hydrate represented by Al₂O₃·nH₂O (n = 1-1.5). Boehmite is preferably used in the form of a boehmite sol. Alumina hydrates having a primary particle diameter (hereinafter particle diameter is referred to as particle size) in a coating solution, or, if aggregated, having a secondary aggregated particle size of from 100 to 300 nm, are preferred.

The inorganic particles may be silica alumina composite particles. Silica alumina composite particles are used preferably in the form of a sol having aggregated particles containing silica and alumina as colloidal particles dispersed in an aqueous medium. They may be silica-alumina composite particles having silica cores and alumina surfaces as well as particles made solely of alumina.

The porous layer of the present substrate may contain a binder or the like, in addition to inorganic particles. In general, as the binder, a water-soluble polymer or an alcohol-soluble polymer or a mixture thereof such as starch or a modified product thereof, polyvinyl alcohol (PVA) or a modified product thereof, a styrene butadiene rubber (SBR) latex, an acrylnitrile butadiene (NBR) latex, gelatin, methylcellulose, ethylcellulose, carboxymethylcellulose, hydroxycellulose, hydroxymethylcellulose, polyvinyl pyrrolidone, polyacrylic acid or polyacrylamide may be used. Among them, PVA is preferably used because sufficient mechanical strength can be obtained without substantially impairing favorable physical properties of boehmite. A binder is preferably contained in an amount of from 1 to 30 parts by weight, particularly from 3 to 15 parts by weight, in relation to 100 parts by weight of inorganic particles. The porous layer of the present substrate preferably has a pore volume of from 0.1 to 5 cm³/g, particularly from 0.3 to 2 cm³/g, more particularly from 0.5 to 1.5 cm³/g, especially particularly from 0.7 to 1.2 cm³/g.

The porous layer of the present substrate preferably has an average pore radius of from 1 to 100 nm, more preferably from 3 to 25 nm in view of absorption of DNA fragments. Further, it is preferred that substantially all the pores fall within the pore radius range of from 1 to 100 nm, with no pores having pore radii larger than 100 nm. Herein, pore characteristics are measured by the nitrogen absorption-desorption method.

The thickness of the porous layer of the present substrate may be appropriately selected and is preferably from 1 to 100 µm after drying. When the inorganic particles are boehmite particles, the thickness is preferably from 10 to 30 µm, particularly preferably from 12 to 25 µm. When the inorganic particles are silica particles, the thickness is preferably 15 to 60 µm, particularly preferably from 20 to 45 µm.

The porous layer may be formed by coating, for example, by adding a binder or the like to inorganic particles and applying the resulting slurry with a roll coater, an air-knife coater, a blade coater, a rod coater, a bar coater, a comma coater, a gravure coater, a die coater, a curtain coater, a spray coater, a slide die coater or the like and drying it, though there are no particular restrictions. In the coating procedure, a coating solution containing inorganic particles is prepared, and the coating solution is applied with coating equipment and dried to make a porous layer. The amount of the coating is preferably from 1 to 100 g/m².

DNA probes may be immobilized on the present substrate preferably by any method without particular restrictions, but ink jetting is preferred.

Now, the present invention will be described with reference to Examples and Comparative Examples.

### EXAMPLE 1 (Example)

A 2 L glass reactor (separable flask equipped with a stirrer and a thermometer) was loaded with 900 g (50 moles) of water and 751 g (12.5 moles) of isopropyl alcohol and heated in a mantle heater to a liquid temperature of 75°C. 204.25 g (1 mole) of aluminum isopropoxide was added with stirring, and hydrolysis was carried out for 120 hours at a constant liquid temperature of from 75 to 78°C. Then, the temperature was elevated to 95°C while the isopropyl alcohol was distilled off, and after addition of 6 g (0.1 mole) of acetic acid, deflocculation was carried out for 48 hours at a constant temperature of from 95 to 97°C. The resulting sol had a solid content of 15 mass% (hereinafter referred simply to as %).

The resulting boehmite sol was mixed with 10 mass% of polyvinyl alcohol (saponification value 99.8%, polymerization degree 4000) based on boehmite, on a solid basis, and applied onto a glass slide, 76 mm x 26 mm x 1 mm thickness (manufactured by Matsunami Glass, made of soda-lime glass) and dried at 70°C to give a biochip substrate. The porous layer (the coating layer) was 16 µm thick, after drying.

DNA fragments (75-mer) linked to fluorophor labels (Cy3, Cy5) were diluted with distilled water to make DNA dilutions ranging from 0.1 to 1 µg/µL. The dilutions were picked up onto the tip of a stainless steel pin having a diameter of 0.5 mm and spotted onto the biochip substrate obtained, about from 0.5 to 1.5 nL for each spot. The substrate was left still until the spots dried, to make a test sample.

### EXAMPLE 2 (Comparative Example)

The procedure in Example 1 was followed except that a glass slide having a poly-L-lysine coated glass slide was used instead of the glass slide having a boehmite porous layer.

### Tests and test results

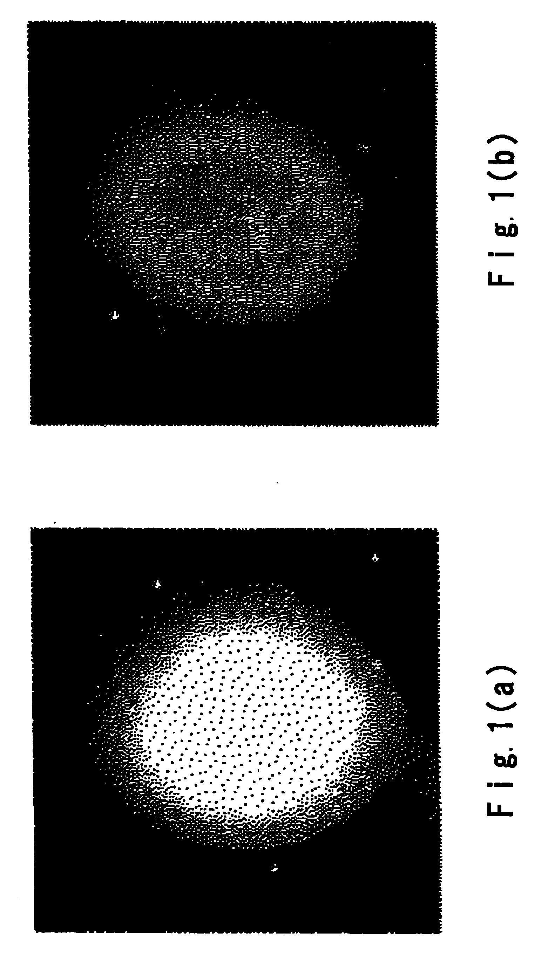

The test samples obtained in Examples 1 and 2 were tested for fluorescence characteristics. The fluorescence at 575 nm and 670 nm from the spotted surfaces of the test samples were observed with a fluorometer under excitation lights of 532 nm and 635 nm. The results are shown in Figs 1 to 3.

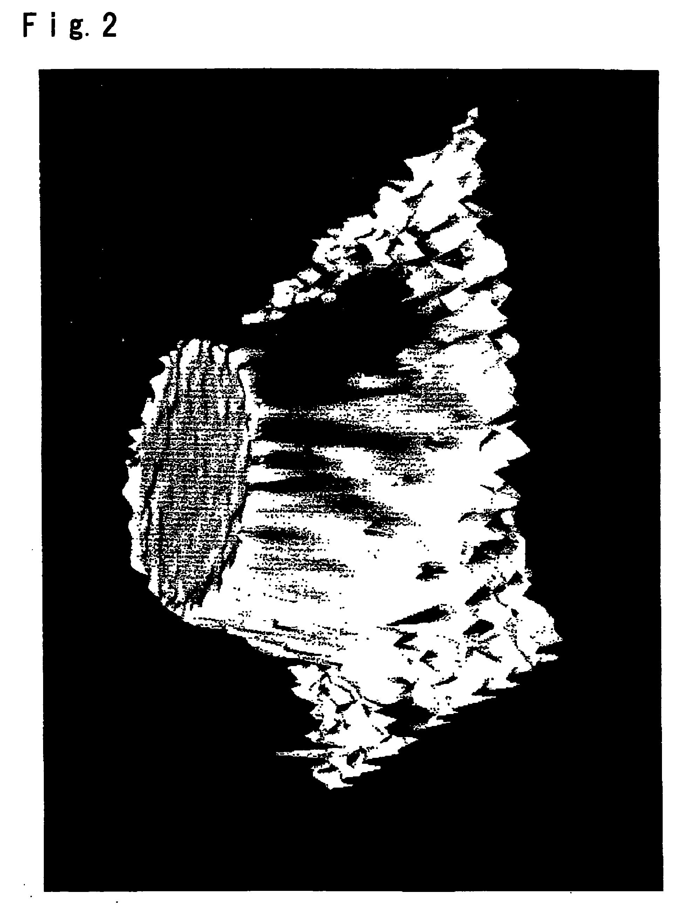

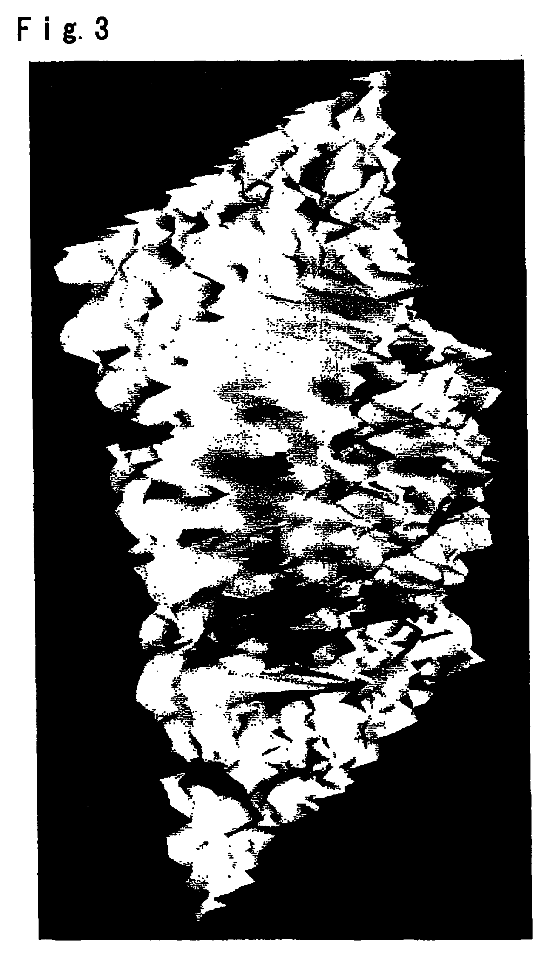

Fig. 1 is a two-dimensional illustration of the fluorescence from a spot. Fig. 1(a) shows the fluorescence from a spot (almost elliptical in shape) deposited on the test sample prepared in Example 1, and Fig. 1(b) shows the fluorescence from a spot deposited on the test sample prepared in Example 2, on the basis that a brighter spot emits stronger fluorescence, and a darker spot emits weaker fluorescence. Stronger fluorescence was emitted from all over the surface of the spot in (a) than in (b). Fig. 2 is a three-dimensional illustration of Fig. 1(a), and Fig. 3 is a three-dimensional illustration of Fig. 1(b).

The spots deposited on the poly-L-lysine coated glass slide (hereinafter referred to as the comparative substrate) emitted weaker and less homogeneous fluorescence (Fig. 1(b) and Fig. 3), while the spots deposited on the glass slide of the present invention having a porous layer containing boehmite emitted almost homogeneous and stronger fluorescence (Fig. 1(a) and Fig. 2). Though the data are not shown, almost no difference was observed in fluorescence characteristics among the spots deposited on the boehmite-coated substrate, whereas difference was observed among the spots deposited on the comparative substrate in fluorescence characteristics, especially in fluorescence intensity.

### INDUSTRIAL APPLICABILITY

The present substrate immobilizes DNA probes firmly at high density with high precision, namely allows efficient and effective hybridization with fluorescently labeled DNA analytes and thereby improves detection sensitivity. Especially, when the porous layer of the present substrate contains boehmite, it fixes negatively charged chemical substances and proteins well and, therefore, is suitable for stamping by ink jetting.

## Claims

1. A biochip substrate comprising a base and a porous layer containing inorganic particles provided on the base.

2. The biochip substrate according to Claim 1, wherein the inorganic particles are alumina particles and/or silica particles.

3. The biochip substrate according to Claim 2, wherein the inorganic particles are boehmite particles.

4. The biochip substrate according to Claim 1, 2 or 3, wherein the porous layer has an average pore radius of from 1 to 100 nm.

5. The biochip substrate according to Claim 1, 2, 3 or 4, wherein the porous layer has a pore volume of 0.1 to 5 cm³/g.

6. The biochip according to Claim 1, 2, 3, 4 or 5, which has a layer containing a DNA fragment on the surface of the porous layer.
